# EUROPEAN PATENT SPECIFICATION

(11) **EP 3 002 591 B1**
(45) Date of publication and mention of the grant of the patent: **06.05.2020**
(21) Application number: 15194046.7
(22) Date of filing: 30.03.2010
(51) Int. Cl.: G01N 33/86, G01N 33/543, G01N 33/49, C12Q 1/56

(54) **COMPOSITION FOR THE DETERMINATION OF COAGULATION CHARACTERISTICS OF A TEST LIQUID**
ZUSAMMENSETZUNG ZUR BESTIMMUNG DER KOAGULATIONSEIGENSCHAFTEN EINER TESTFLÜSSIGKEIT
COMPOSITION POUR LA DÉTERMINATION DES CARACTÉRISTIQUES DE COAGULATION D'UN LIQUIDE D'ESSAI

(43) Date of publication of application: 06.04.2016
(62) Divisional of application: 10711886.1
(73) Proprietor: C A Casyso AG, 4052 Basel (CH)
(72) Inventor: SCHUBERT, Axel, 81927 München (DE)
(74) Representative: Isarpatent

(56) References cited:
- EP-A1- 1 234 614
- WO-A1-96/32497
- WO-A1-2008/093216
- WO-A2-2009/115608
- US-A- 4 755 461
- DICKINSON C D ET AL: "Identification of surface residues mediating tissue factor binding and catalytic function of the serine protease factor VIIa", PROCEEDINGS OF THE NATIONAL ACADEMY OF SCIENCES, NATIONAL ACADEMY OF SCIENCES, US, vol. 93, 1 December 1996 (1996-12-01), pages 14379-14384, XP002267001, ISSN: 0027-8424, DOI: 10.1073/PNAS.93.25.14379

## Description

### FIELD OF THE INVENTION

The present invention is directed to diagnostic compositions for use in the viscoelastic analysis of a test liquid being a whole blood or blood plasma, and to a container comprising same. The present invention further is directed to a method of performing a viscoelastic analysis on a test liquid being a whole blood or blood plasma, and to the use of the diagnostic composition in such a method.

### BACKGROUND

The coagulation of blood is a complex process during which blood forms solid clots. It is an important part of hemostasis (the cessation of blood loss from a damaged vessel) whereby a damaged blood vessel wall is covered by a blood clot to stop hemorrhage and aid repair of the damaged vessel. Disorders in coagulation can lead to increased hemorrhage and/or thrombosis and embolism.

In a normal individual, coagulation is initiated within 20 seconds after an injury occurs to the blood vessel damaging the endothelial cells. Platelets immediately form a haemostatic plug at the site of injury. This process is called primary haemostasis. Secondary haemostasis follows if plasma components called coagulation factors respond in a complex cascade to form fibrin strands which strengthen the platelet plug.

The coagulation cascade of secondary hemostasis has two pathways, the Contact Activation pathway (formerly known as the Intrinsic Pathway) and the Tissue Factor pathway (formerly known as the Extrinsic pathway) that lead to fibrin formation. It was previously thought that the coagulation cascade consisted of two pathways of equal importance joined to a common pathway. It is now known that the primary pathway for the initiation of blood coagulation is the Tissue Factor pathway. The pathways are a series of reactions, in which a zymogen of a serine protease and its glycoprotein co-factor are activated to become active components that then catalyze the next reaction in the cascade. Coagulation factors are generally indicated by Roman numerals from I - XIII, with a lowercase 'a' appended to indicate the activated form.

Fibrinolysis is the process where the fibrin clot is broken down. Tissue plasminogen activator (tPA) and urokinase are the agents that convert plasminogen to active plasmin, thus allowing fibrinolysis to occur.

The detection of normal or decreased functionality of these coagulation components is important in order to assess patients' hemostasis disorders.

Several methods of measuring the coagulation characteristics of blood are known. Some such devices attempt to simulate the natural flow of blood in the veins and arteries of a living subject, while other measurement techniques are performed in static blood volumes.

An accurate measurement of the ability of a patient's blood to coagulate in a timely and effective fashion is crucial to certain surgical and medical procedures. Rapid and accurate detection of abnormal coagulations is also of particular importance with respect to appropriate treatment to be given to patients suffering from clotting disorders. Often the condition of such patients makes it necessary to administer blood components, anti-coagulants, certain fibrinolytic agents, anti-platelet agents, or compounds inducing the reverse effects of said agents. In these cases, the treatment dose can be adapted to the extent of a clotting disorder previously determined.

Measurements of blood clotting are provided by various devices, for example as disclosed in (US 5,777,215), (US 6,537,819), or (US 5,777,215). These devices measure the mechanical properties of the clot throughout its structural development. These systems are summarized under the term "viscoelastic methods", as they continuously detect viscoelastic properties of the blood clot while its formation and lysis.

A viscoelastic measurement provides information about several distinct parameters, for example the time between coagulation activation and clot initiation (clotting time CT), the dynamics of clot formation (clot formation time CFT), the firmness of the clot (amplitudes A5-A30 and maximum clot firmness MCF), or the extent of fibrinolysis (maximum lysis ML).

A number of references describe instruments for measuring blood clotting characteristics based upon mechanical movements. These instruments monitor the elastic properties of blood as it is induced to clot under a low shear environment, i.e. in static blood volumes. The patterns of change in shear elasticity enable the determination of the kinetics of clot formation, as well as the strength and stability of the formed clot. The strength and stability of the clot provide information about the ability of the clot to perform the "work of hemostasis" (i.e., stop or prevent abnormal bleeding) and about the adequacy of blood platelet-fibrin interaction. The kinetics of clot formation mainly provides information about the functionality of coagulation factors. Analysis of all of this information provides results which are useful to predict bleeding, to monitor and manage thrombosis, or to monitor fibrinolysis.

However, as the clotting process consists of various interlinked components, the use of specific activators and inhibitors is further applied in order to detect hemostasis disorders more specifically.

Accordingly, the reagents used in viscoelastic analysis consist of an initial activator (e.g., an activator of either the intrinsic or the extrinsic pathway) and optionally one or more inhibitors (e.g., fibrinolysis inhibitors, heparin inhibitors, platelet inhibitors) and/or one or more further specific factor of the coagulation cascade.

Optionally further components may be added:
- Calcium (CaCl₂): The calcium is added for recalcification of the sample. Blood samples can be prevented from clotting by several different anticoagulatory substances like heparin, EDTA, citrate. Typically functional tests are done with blood anticoagulated with citrate. Citrate moderately complexes calcium of the blood sample. Calcium is necessary for the coagulation process, it is involved in complex formation and is co-factor for most of the coagulation factors (e.g., FI, FII, FV, FVII, FVIII, FIX, FX, FXI, FXIII, TF). Therefore, recalcification of the sample is necessary to ensure correct coagulation in the sample, if the sample was citrated during blood withdrawal (by using a blood tube containing citrate).
- Phospholipids: Several complexes in the coagulation cascade are phospholipid-dependent and, therefore, additional phospholipids might be added.
- Stabilizers: For the stabilization of the reagents between the time of production and the analysis (e.g. albumin, gelatine)

Depending on the diagnostic aim, these reagents can be used either alone or in combination: For example, a measurement with only intrinsic activator in the sample can be combined with a measurement with intrinsic activator and a sufficient amount of heparin inhibitor (e.g., heparinase) in the sample to detect the presence of heparin in the test liquid; a combination of extrinsic activator and platelet inhibitor (e.g., Cytochalasin D) in the sample is applied to determine the activity of fibrinogen without platelet contribution in the test liquid.

There is a reagent concept for viscoelastic measurements in the literature (ReoPro-modified TEG: Wenker et al.: Thrombelastography, The Internet Journal of Anesthesiology, 2000, Volume 1 Number 3; (http://www.ispub.com/ostia/index.php?xmlFilePath=journals/ija/ volln3/teg.xml); Ruttmann et al.: Hemodilution Enhanced Coagulation Is Not Due to Platelet Clumping, Anesthesiology 2004; 101: A150; Recombiplastin- and ReoPro-modified TEG:http://www.transfusionguidelines.org.uk/ docs/pdfs/bbt_app-use_teg-sop-example.pdf; TF- and Trasylol-modified TEG: Tanaka et al.: Evaluation of a novel kallikrein inhibitor on hemostatic activation in vitro, Thrombosis Research, Volume 113, Issue 5 , 2004, Pages 333-339) that is based on the combination of commercially available activator reagents intended for other tests, such as the prothrombin time activator Innovin or Recombiplastin®, combined with customer-made CaC12 solution and drugs, such as ReoPro® (abciximab) and Trasylol® (aprotinin). This leads to a low standardization, many pipetting steps and many sources of error.

There is a reagent system for viscoelastic measurements marketed by Pentapharm, which is based on standardized reagents, most of which are provided to the customer in a liquid form, which are pipetted by the user into the test cup using standardized operating procedures. This standardizes the application; however it still requires several pipetting steps for the analysis. For example, to perform a FIBTEM test together with an EXTEM control test, the pipetting of blood, CaCl₂ solution, extrinsic activator and a platelet inhibitor may result in the performance of a total of 8 pipetting steps (including three times changing of the tip during one test procedure) and the need for 3 different reagents that have to be handled by the user. This provides a requirement for training consumes time and is a potential source of error.

There are other reagent systems on the market, which are based on a variety of reagents. Some of them are liquid, and have to be pipetted into the cup (e.g. CaCl₂ solution), some are dried into the test cup (such as heparinase) and some are provided in small vials, in a quantity intended for one test. A characteristic of these reagents is that still each reagent is typically provided alone, and therefore several steps are required at least for tests requiring more than one active reagent.

Accordingly, some efforts have been done in the past to provide a simpler reagent system for viscoelastic measurements of blood or blood components:
- drying them directly into the cup which takes the volume of the sample during measurement
- compound stable liquid combinations of the reagents in the working concentration

One shortcoming of the strategy of drying active reagents directly into the test cup is that these are typically made of plastic and are light, which makes the filling of these cups in automated reagent dispensing lines more difficult. This makes manual filling steps necessary or the development of specialized equipment, which are both costly. Another shortcoming of this strategy is that the active components or stabilizers may interfere with the adhesion strength of the blood clot on the cup surfaces, which is required to perform correct measurements. For example, there has been shown diminished clot adhesion after incubation of albumin solution into the cup (Albumin is a typical stabilizer used to stabilize all kinds of proteins in reagents):

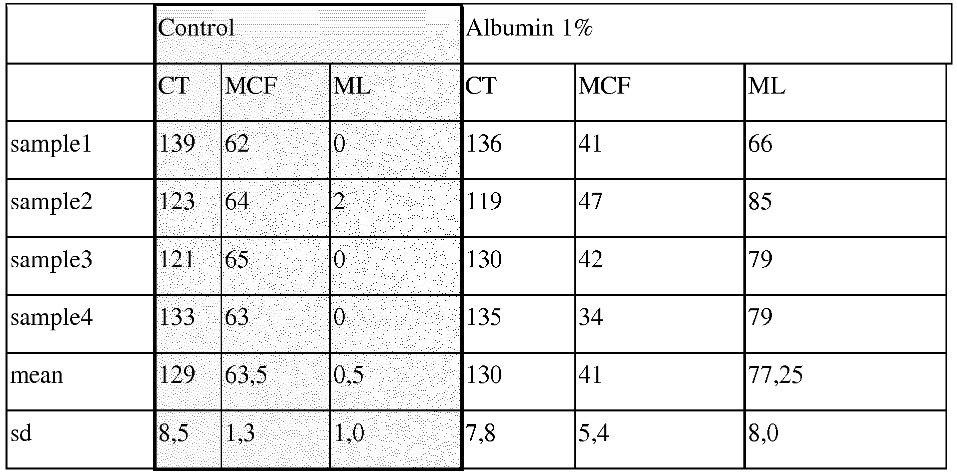

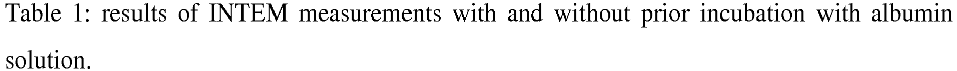

Another possible strategy to simplify the handling of the reagents is to combine the different reagents necessary for one test in liquid phase in their working concentration. The main problem here is the interaction of the different substances while staying together for a longer period. Some components negatively affect the stability of each other when staying together in the liquid phase at higher concentrations; for example, CaCl₂ disturbs the stability of Tissue Factor reagent in liquid phase over the time.

Moreover, if these combined reagents should be provided in an amount sufficient for exactly one test, another problem would arise: In this case, the very small portion of liquid reagent might stick to parts of the reagent container or the cap and might thus not mix sufficiently with the test liquid when the analysis is performed.

One strategy proposed to solve the named aspects was disclosed by Kolde et al. in US patent application 20040071604. In this application, a cup system for viscoelastic analyses is presented, in which the lower end of the cup is divided in several reagent chambers. This allows to place the reagents independently into the different chambers, without mixing them and then to freeze-dry the reagents.

However, disadvantages of this solution include the need for a very precise pipetting process, as the separate reagent chambers are very small and also the problem that the reagent drops might still mix before the freeze-drying by vibrations present on the reagent filling line. Another problem is the possible air-drying of the small reagent drops during the processing under room conditions before the lyophilisation process starts. Again the problem of automatically handling the small plastic and thus very light using standard reagent-filling lines is present.

The most coagulation test methods in routine lab use just measure the time from adding an activator to the sample until the first initial formation of a fibrin clot can be detected (= clotting time). They stop at this point and no further measurement is done. This has the implications, that in these methods a firm adhesion of the blood to the surfaces of the measurement cell is not necessary. Accordingly, the variety of analyzers and reagents available for the assessment of clotting times with such methods does not have to handle the unique problems connected with viscoelastic measurements.

EP 0 972 203 describes a reagent for determining aPTT, comprising at least one coagulation protein, phospholipids and an activator of intrinsic coagulation in co-lyophilized form. However, here the problem arises that all components required for performing the analysis are in immediate contact with each other. Therefore, problems regarding long-term stability or incompatibilities may occur by using substances in this form.

WO2008093216 discloses diagnostic compositions, containers, method and uses for performing one single viscoelastic measurement lacking however the aspect of providing the reagents in spatially separated form.

US4755461 teaches to incorporate tissue factor and CaC12 into different tablets, since these reagents were found to be incompatible even when provided in dry form in a mixture.

### SUMMARY OF THE INVENTION

Therefore, it is an object of the present invention to provide a diagnostic composition, which allows for a safe, reproducible and easy to use procedure for different tests in viscoelastic systems. It is a further object of the present invention to provide a diagnostic composition which is specifically adapted to one single analysis of a blood sample and has a superior reagent stability regarding prior art compositions. It is a still further object of the present invention to provide a diagnostic method which provides reliable and reproducible results, is easy to handle and which provides a standardized system for the determination of the coagulation characteristics of a blood sample. A further object of the invention is to provide a diagnostic composition of the above kind, having an improved long-term stability.

These objects are attained by the diagnostic composition of claim 1, the container of claim 9, the method of performing a viscoelastic analysis on a test liquid of claim 12 and the use of claim 14.

Preferred embodiments are set forth in the dependent claims.

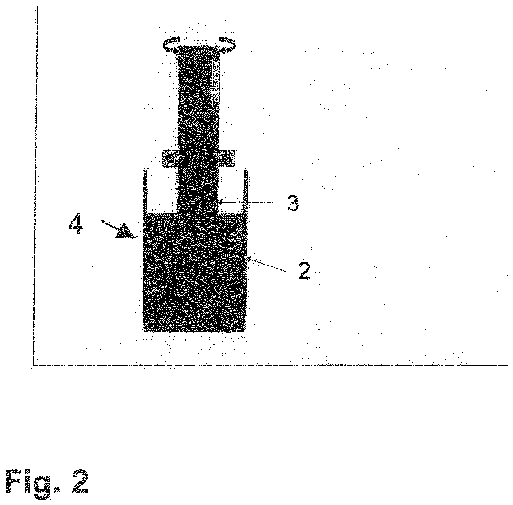

By using the diagnostic composition of the invention, the tests may be performed as follows: a defined volume of a sample, namely whole blood or blood plasma, is added directly into a container 1 containing the diagnostic composition. After dissolving of the composition in the blood sample, the resulting mixture is pipetted from the container 1 into the measuring cup 2 of a measuring apparatus 4. The cup 2 is then put into a position such that the pin 3 is immersed into the liquid in the test cup (cp. Fig. 2).

Therefore, the user needs only 4 pipetting steps for each test to perform (in the prior art liquid system up to 8 steps, see above) and no change of the pipette tip is necessary.

Thus, it is clear that the present system for the determination of coagulation characteristics of a blood sample can be handled easier, thereby making the likelihood of errors smaller, which can be due to an imprecise line of action by a (potentially less experienced) operator.

According to the invention, the constituents are not present in a substance mixture, but in spatially separated form, wherein the constituents are each incorporated in spatially separated pellets.

This allows for an improved long-term stability of the composition without deteriorating its other characteristics.

Therefrom, further advantages may arise as, for example, a higher reproducibility of the results to be achieved, and thus, a higher degree of standardization.

### BRIEF DESCRIPTIONS OF THE DRAWINGS

Further features and advantages of the present invention will be evident from a description of embodiments with reference to the figures.

In the figures:

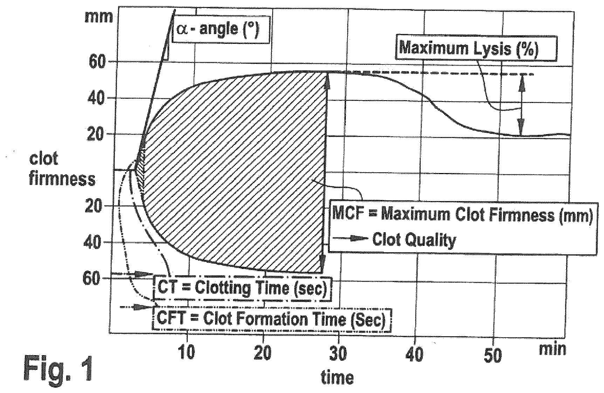

Figure 1 is an exemplary diagram showing a typical thrombo-elastometric measurement;
Figure 2 is showing a measuring apparatus 4 for thromboelastometric analysis;
Figure 3 is an illustration of a measuring cup 2 of a measuring apparatus 4 of the prior art;
Figure 4A, B, C are schematic cross-sectional views of three preferred embodiments of a container 1 of the present invention.

### DETAILED DESCRIPTION OF THE INVENTION

In a first aspect, the present invention provides a diagnostic composition for use in the viscoelastic analysis of a test liquid selected from whole blood or blood plasma, comprising:
a) at least one activator of coagulation selected from tissue factor (TF);
b) a calcium salt, preferably CaC12, in an amount sufficient to ensure recalcification of the test liquid; and,
c) one or more inhibitors of heparin, platelets or fibrinolysis;
characterized in that
the constituents are present in an essentially dry form and in an amount sufficient for performing one single viscoelastic analysis of a specified test liquid,
and wherein the constituents are not present in a substance mixture, but in spatially separated form, wherein the constituents are each incorporated in spatially separated pellets.

The calcium salt preferably is CaCl₂.

The diagnostic composition or reaction mixture of the present invention comprises constituents, which are per se known in the art. One difference to the prior art approaches is, however, that the mixture of these constituents is provided in an essentially dry form.

"Essentially" dry in the context of the invention means a state, wherein the mixture is essentially free from any liquid or moisture, in particular being depleted of water. Water or any other liquid, however, may be present as residue in the mixture, but only to an extent, which does not negatively influence the stability of the overall composition. In particular, it has to be excluded that an interaction occurs between the different constituents, which negatively affects the stability. A remaining amount of liquid, preferably water, in the composition of up to 10 % by weight should be acceptable.

The amount sufficient for performing one single viscoelastic analysis of a specified test liquid, for example a blood sample, is that amount of all constituents in mixture, which provides the required concentration of the reagents in the final analysis of the coagulation characteristics of the blood sample, i.e. in the cup 2 of a measuring apparatus 4. Therefore, it is not necessary to further portion the diagnostic composition before or after dissolving it in a liquid.

Further, this is achieved by dissolving the substances in the test liquid (whole blood or blood plasma) itself, not by dissolving the constituents in an amount of liquid diluent leading to the final working concentration.

It is an important element of the present invention that the constituents are not present in a substance mixture, but in a spatially separated form. This avoids stability related problems.

The spatial separation of the constituents is realized by incorporating each constituent into a separate carrier material. This term means that the constituents are each incorporated in spatially separated entities, namely pellets. The term "separate carrier material" does not necessarily mean that the materials used are chemically different. It is only meant that they are spatially separated from each other.

The carrier takes the form of pellets. Those pellets may be produced by methods which are per se known in the prior art. However, it is preferred to use one of the both methods of producing pellets, which will be shortly outlined in the following:
a) The respective constituent in solution is mixed with a suitable carrier material, which mixture is then converted into spray form. This spray, containing droplets of the mixture, is shock frozen in liquid nitrogen, will be warmed then under a protective atmosphere and is stored in an air tight container (such as a vial).
b) Pellets are produced such as in a), however, the starting mixture does not contain any constituent. The constituent is provided as a solution and is allowed to drop onto the pellets under a protective atmosphere. After being dried onto the pellets, the pellets are packaged in an air-tight container.

In a preferred embodiment, the carrier material comprises at least a carbohydrate, e.g. saccharose, lactose or cellulose. The carrier material preferably shows no significant influence on the clotting behaviour, the clot formation behaviour, or the clot lysis behaviour.

Activators of coagulation include intrinsic and/or extrinsic activators.

The extrinsic activator of coagulation is the Tissue Factor (TF) and is more preferably selected from lipidated TF or rTF.

Intrinsic activators of coagulation include celite, ellagic acid, sulfatit, kaolin, silica, RNA, or mixtures thereof.

As a second feature, the diagnostic composition of the present invention comprises a calcium salt such as CaCl₂, wherein CaCl₂ is preferably present in an amount of about 1-100 µmol/ml of test liquid. As mentioned above, the amount of CaCl₂ must be sufficient to ensure recalcification of the test liquid, namely whole blood or blood plasma, if the sample was decalcified before. It turned out that the amount of from 3-30 µmol/ml is optimal to achieve this requirement. In order to determine the required amount of CaCl₂ to be contained in the diagnostic composition even more precisely, the exact volume of the test liquid to be collected from the patient has to be known as well as the amount of decalcifying reagent employed.

The diagnostic composition of the present invention contains one ore more inhibitors, being selected from one or more of a platelet inhibitor, fibrinolysis inhibitor, or heparin inhibitor.

Those inhibitors may be used and combined depending on the precise diagnostic demands, for example, the platelet inhibitor may be a cyto-skeletton inhibitor or a GPIIb/IIIa antagonist. The like, the fibrinolysis inhibitor can be selected, for example, from aprotinine, tranexamic acid, or eaca; the heparin inhibitor might be selected, for example, from heparinase, protamine or protamine-related peptides. A coagulation factor can be selected, for example, from one or more coagulation factors or activated coagulation factors preferably FXa or FVa or activated protein C or FVIIa. However, it is noted that this is only a preferred selection and further inhibitors can be used if required.

In a preferred embodiment, the diagnostic composition may also contain one or more stabilizers, wherein the stabilizer preferably is albumin or gelatine.

In a preferred embodiment, the diagnostic composition may also contain one or more phospholipids, wherein the phospholipids may be a composition of different phospholipids like phosphatidyserine, phosphatidylethanolamine and phosphatidylethanolcholine. For example, mixtures of phospholipids extracted from rabbit brain may be used.

The present diagnostic composition may have the following constitution in preferred embodiments:
- extrinsic activation insensitive for heparin: Combination of extrinsic activator tissue factor heparin inhibitor and stabilizer and CaCl₂
- extrinsic activation without platelet activation: Combination of extrinsic activator tissue factor, platelet inhibitor and stabilizer and CaCl₂
- extrinsic activation without platelet activation, insensitive for heparin: Combination of extrinsic activator tissue factor, platelet inhibitor, heparin inhibitor and stabilizer and CaCl₂
- extrinsic activation with inhibition of fibrinolysis: Combination of extrinsic activator tissue factor, fibrinolysis inhibitor and stabilizer and CaCl₂
- extrinsic activation with inhibition of fibrinolysis, insensitive for heparin: Combination of extrinsic activator tissue factor, fibrinolysis inhibitor, heparin inhibitor and stabilizer and CaCl₂
- extrinsic activation with additional coagulation factor, insensitive for heparin: Combination of extrinsic activator tissue factor, one additional coagulation factor, heparin inhibitor and stabilizer and CaCl₂

In a second aspect, the present invention provides a container according to claim 9 (1), comprising the diagnostic composition as defined above. The container 1 preferably takes the form of a vial or a cuvette. The container 1 is formed from a material (e.g., plastic or glass) which is not corroded or otherwise affected by the reagents to be filled in or the test liquid to be filled in.

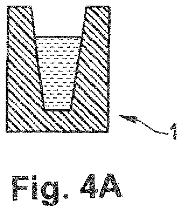

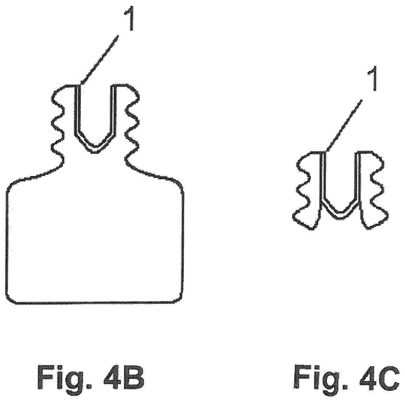

The container 1 may have cylindrical shape, but its shape does not necessarily have to be cylindrical. The container 1 may have a form which reduces its inner lateral profile from the upper opening to the bottom, as for example a conically shaped form as indicated in Fig. 4 or at least a partially conical form. This provides a better handling of the usually small amounts of liquid reagent mixture. Using flat bottom vials with a smaller diameter would reduce this problem, but such vials might then have an opening diameter which is too narrow to be handled with standard pipetting equipment used in connection with diagnostic devices like, for example, the ROTEM thrombo-elastometer. (Regarding the design of the ROTEM system and how to use it, it is referred to the publication of US 5,777,215.)

Furthermore, such small vials might become harder to manage for common automated processing systems and might thus increase the production costs.

The cross-section of a basically axially symmetric container 1 of a preferred embodiment is shown in Fig. 4A. However, it should be explicitly noted that the present invention is not restricted thereto, and also U-shaped, rectangular shaped or the like forms may be used.

As mentioned above, the container 1 may be closed or sealed by a lid 5 or the like in order to avoid a loss of reagents, or an invasion of contaminants, water etc.

In a further embodiment, the container 1 is designed in a way that it can be directly used as the measuring cup 2 of the viscoelastic measuring apparatus 4. In other words, a viscolelastic analysis can be performed such that a respective container 1 is provided, the test liquid is added into the container 1, and the measurement is performed directly in the container 1. In this case, only the blood dispension into the container has to be performed as a liquid transfer step, which can be realized by using a manual pipette, an automated pipette, an automated dispenser or any other liquid transfer equipment.

In this embodiment, the container 1 might be designed by combination of two materials, e.g., glass and plastic or glass and a surface covering. In this context the part of the container made from glass does not necessarily have to clasp the entire underside of the plastic part but might be constructed according to Figure 4C.

In Figure 4B and C, a further embodiment of the invention can be seen. The container 1 (for example a cuvette) may be incorporated in a larger structure, for example a glass article, which provides some technical advantages: this set-up may provide a holding for the container.

In these embodiments, possible coagulation activation in the test liquid by the glass surface is excluded, while the superior sealing properties of the glass material when compared to plastic material are still used. The similar effect of suppressing possible coagulation activation in the test liquid by the glass surface can be realized by covering the glass surface (or at least the inner portion of the glass surface) with a layer of one or more substances that are not able to activate coagulation if they are in contact with blood or blood components.

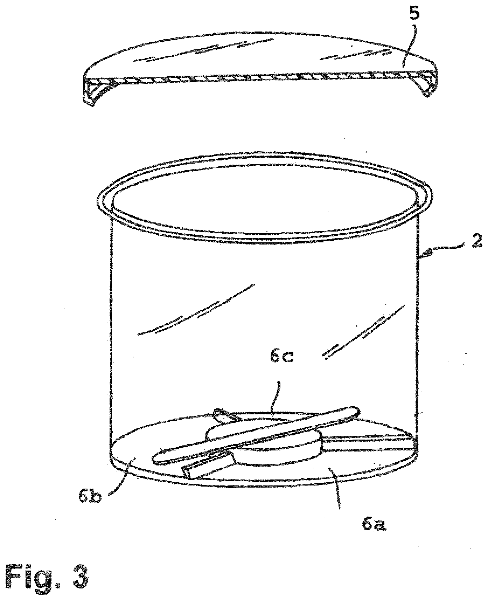

For comparison, a prior art measuring cup 2 according to US 2004/0071604 is illustrated in Fig. 3:
A container 1 is provided, which serves as reagent support and measuring vessel (i.e. can be regarded as a measuring cup 2) for analysis using various analytical processes, and has a region which is divided into at least two chambers (6a, 6b, 6c) by one or more bars extending from the container wall or the container base, wherein the chambers are arranged so that liquid or solid reagents may be introduced therein without them being able to be mixed by diffusion or running into one another. The container 1 is used for drying or freeze-drying by with completely or partly filled chambers and serves at the same time as a measuring vessel after re-dissolving the dried material by adding water, reagent solution, or the sample present in aqueous phase.

In a third aspect, the present invention is directed to a method of performing a viscoelastic analysis on a test liquid selected from whole blood or blood plasma, comprising the steps of:
a) providing a test liquid selected from whole blood or blood plasma;
b) providing a container 1 according to one of claims 9-11;
c) adding the test liquid into said container 1, thereby dissolving the diagnostic composition contained therein;
d) transferring the mixture of said test liquid and said diagnostic composition into a cup 2 and put it into an apparatus 4 suitable for performing a viscoelastic analysis,
   or
   putting the container 1 into an apparatus 4 suitable for performing a viscoelastic analysis; and
e) performing the viscoelastic analysis of said mixture.

As already mentioned above, the test liquid is whole blood or blood plasma, preferably mammalian, more preferably human whole blood or blood plasma.

Step c) of the method of the present invention preferably takes about 1-60, more preferably 2-10 sec and most preferred is about 5 sec. Following that time, the mixture of the diagnostic composition and the blood sample should be quickly transferred to the measuring cup 2 of the measuring apparatus 4. This is done in step d) by manually or automatically pipetting the mixture from the container 1 and by transferring it thereby to the apparatus 4, i.e. to the measuring cup 2 of the apparatus 4.

As an alternative, if the container 1 of the present invention is the measuring cup 2, the measurement is performed directly in the container 1. In this case, step d) may be omitted. The apparatus 4 preferably is a device suited for viscoelastic measurements, for example devices disclosed in (US 5,777,215), (US 6,537,819), or (US 5,777,215).

One example of that apparatus 4 is shown in Fig. 2:
After the formation of the clot between cup 2 (cuvette) and pin 3, the clot itself is stretched by the movement of the pin 3 relative to the cup 2. The detection of the characteristic parameters of the clot is based on the mechanical coupling of cup 2 and pin 3 by the clot. This is only possible if the clot adheres on the surfaces of both cup 2 and pin 3. So, a firm adhesion on the surfaces of both cup 2 and pin 3 is essentially required for the viscoelastic analysis.

The method of the present invention comprises such a viscoelastic analysis of a blood sample in order to determine its coagulation characteristics, wherein such a viscoelastic analysis in the broadest sense is the measurement of a relative movement of a cuvette containing a blood sample relative to a punch. The analysis preferably comprises the determination of the clotting time, the clot formation time, and the firmness of the clot over time including fibrinolytic effects.

In practice, the following steps may be performed:
1. a defined volume of a sample, namely whole blood or blood plasma, is added directly into a vial containing the reagent composition; the measurement should start at a time close to the moment of adding the sample
2. after dissolving of the reagent mixture in the sample (5 sec.) the reagent-sample mixture is pipetted from the reagent vial into the measuring cup 2 (not necessary if the vial functions itself as measuring cup 2)
3. the cup 2 is then put into a position such that the pin 3 is immersed into the liquid contained in the test cup, the measurement continues until stopped by the user

Therefore, the user needs not more than 4 pipetting steps in total for each test to perform (compared to the up to 8 steps required for a liquid reagent system) and no change of pipette tip is necessary when preparing one test. This clearly indicates the direct benefit of the present invention for the person who is performing such tests.

In a further aspect, the present invention is directed to the use of a diagnostic composition or a container 1 according to claim 14 in a method for analyzing the viscoelastic behaviour of a test liquid selected from whole blood or blood plasma.

Although the present invention has been described in accordance with preferred embodiments, it is obvious for a person skilled in the art that modifications are possible in all embodiments.

### Reference signs

- 1: container
- 2: measuring cup
- 3: pin
- 4: measuring apparatus
- 5: lid
- 6 a,b,c: reagent chambers

## Claims

1. A diagnostic composition for use in the viscoelastic analysis of a test liquid selected from whole blood or blood plasma, comprising:
a) at least one activator of coagulation selected from tissue factor (TF);
b) a calcium salt, preferably CaCl₂, in an amount sufficient to ensure recalcification of the test liquid; and,
c) one or more inhibitors of heparin, platelets or fibrinolysis;
**characterized in that**
the constituents are present in an essentially dry form and in an amount sufficient for performing one single viscoelastic analysis of a specified test liquid,
and wherein the constituents are not present in a substance mixture, but in spatially separated form, wherein the constituents are each incorporated in spatially separated pellets.

2. The composition according to claim 1, where the spatial separation of the constituents is realized by incorporating each constituent into a separate carrier material, where the carrier material preferably comprises at least a carbohydrate, e.g. saccharose, lactose or cellulose.

3. The composition according to claim 2, where the carrier material shows no significant influence on the clotting behaviour, the clot formation behaviour, or the clot lysis behaviour.

4. The diagnostic composition of claim 1, wherein the Tissue Factor is selected from lipidated TF or rTF.

5. The diagnostic composition of claim 1, wherein platelet inhibitor is a cyto-skeletton inhibitor or a GPIIb/IIIa antagonist, e.g. Cytochalasin D, or wherein the fibrinolysis inhibitor is selected from aprotinine, tranexamic acid, or eaca, or wherein the heparin inhibitor is selected from heparinase, protamine or protamine-related peptides.

6. The diagnostic composition of one or more of the preceding claims, wherein the further coagulation factor is selected from one or more coagulation factors or activated coagulation factors, preferably FXa or FVa or activated protein C or FVIIa, or wherein CaCl₂ is present in an amount of about 1-100 µmol/ml of the test liquid, preferably the blood sample, or
wherein the dry form is a lyophilized form.

7. The diagnostic composition of one or more of the preceding claims, which further comprises a stabilizer, or
wherein the stabilizer is albumin or gelatine.

8. The diagnostic composition of any one of claims 1-7, further comprising one or more phospholipids selected from phosphatidylserine, phosphatidylethanolamine, phosphatidylethanolcholine and mixtures of phospholipids extracted from rabbit brain.

9. A container, comprising the diagnostic composition of one or more of claims 1-8.

10. The container of claim 9, wherein the container (1) takes the form of a vial or a cuvette, and preferably is formed in a way that the inner lateral profile reduces from the opening to the bottom.

11. The container of claim 9 or 10 with its inner portion shaped in a manner that it can be attached to a device for performing viscoelastic measurements, and preferably is consisting of at least two different materials, where one material forming basically or at least partially the outer shape of the container enables hermetical sealing of the container by a suited cover, while the other material basically covering the portion conceiving the test liquid enables proper adhesion of blood or blood components without activating the coagulation cascade.

12. A method of performing a viscoelastic analysis on a test liquid selected from whole blood or blood plasma, comprising the steps of:
a) providing a test liquid selected from whole blood or blood plasma;
b) providing a container (1) according to one of claims 9-11;
c) adding the test liquid into said container (1), thereby dissolving the diagnostic composition contained therein;
d) optionally transferring the mixture of said test liquid and said diagnostic composition into an apparatus (4) suitable for performing a viscolelastic analysis;
or
putting the container 1 into an apparatus 4 suitable for performing a viscolelastic analysis; and
e) performing the viscoelastic analysis of said mixture.

13. The method of claim 12, wherein the test liquid preferably is a mammalian, more preferably a human blood sample, and/or
wherein step c) takes about 1-60 sec., preferably about 2-10 sec., more preferably about 5 sec, and/or
wherein the mixture is transferred in step d) by manually or automatically pipetting the mixture from the container (1) and by transferring it thereby to the apparatus (4), and/or
wherein the mixture is transferred to the measuring cup (2) of the apparatus (4), and/or
wherein the apparatus (4) is a thromboelastometer or a thrombelastograph, and/or
wherein the analysis comprises the determination of the clotting time, the clot formation time, the firmness of the clot over time and/or fibrinolysis.

14. Use of a diagnostic composition of one or more of claims 1-8 or of a container (1) of one or more of claims 9-11 in a method for analyzing the viscoelastic behaviour of a test liquid selected from whole blood or blood plasma, wherein preferably at least two diagnostic compositions of one or more of claims 1-8 or at least two containers (1) of one or more of claims 9-11 are used in a combined method for analyzing the viscoelastic behaviour of the same test liquid selected from whole blood or blood plasma.

## Patentansprüche

1. Diagnostische Zusammensetzung zur Verwendung bei der viskoelastischen Analyse einer Testflüssigkeit, ausgewählt aus Vollblut oder Blutplasma, enthaltend:
a) mindestens einen Gerinnungsaktivator, ausgewählt aus Gewebefaktor (*tissue factor,* TF);
b) ein Calciumsalz, vorzugsweise CaCl₂, in einer Menge, die ausreicht, um eine erneute Kalzifizierung der Testflüssigkeit sicherzustellen; und,
c) einen oder mehrere Inhibitoren von Heparin, Thrombozyten oder Fibrinolyse;
**dadurch gekennzeichnet, dass**
die Bestandteile in einer im Wesentlichen trockenen Form und in einer Menge vorliegen, die ausreicht, um eine einzelne viskoelastische Analyse einer bestimmten Testflüssigkeit durchzuführen,
und wobei die Bestandteile nicht in einer Substanzmischung vorhanden sind, sondern in räumlich getrennter Form, wobei die Bestandteile jeweils in räumlich getrennten Pellets enthalten sind.

2. Zusammensetzung nach Anspruch 1, wobei die räumliche Trennung der Bestandteile durch Einbau jedes Bestandteils in ein separates Trägermaterial realisiert wird, wobei das Trägermaterial vorzugsweise mindestens ein Kohlenhydrat, zum Beispiel Saccharose, Lactose oder Cellulose enthält.

3. Zusammensetzung nach Anspruch 2, wobei das Trägermaterial keinen signifikanten Einfluss auf das Gerinnungsverhalten, das Gerinnselbildungsverhalten oder das Gerinnsellyseverhalten zeigt.

4. Diagnostische Zusammensetzung nach Anspruch 1, wobei der Gewebefaktor ausgewählt ist aus lipidiertem TF oder rTF.

5. Diagnostische Zusammensetzung nach Anspruch 1, wobei der Thrombozyteninhibitor ein Zytoskelettinhibitor oder ein GPIIb/Illa-Antagonist ist, z. B. Cytochalasin D, oder wobei der Fibrinolyse-Inhibitor ausgewählt ist aus Aprotinin, Tranexamsäure oder Eaca, oder wobei der Heparin-Inhibitor ausgewählt ist aus Heparinase, Protamin oder mit Protamin verwandten Peptiden.

6. Diagnostische Zusammensetzung nach einem oder mehreren der vorhergehenden Ansprüche, wobei der weitere Gerinnungsfaktor ausgewählt ist aus einem oder mehreren Gerinnungsfaktoren oder aktivierten Gerinnungsfaktoren, vorzugsweise FXa oder FVa oder aktiviertem Protein C oder FVIIa, oder
wobei CaCl₂ in einer Menge von etwa 1 bis 100 pmol/ml der Testflüssigkeit, vorzugsweise der Blutprobe, vorhanden ist, oder
wobei die trockene Form eine lyophilisierte Form ist.

7. Diagnostische Zusammensetzung nach einem oder mehreren der vorhergehenden Ansprüche, die ferner einen Stabilisator enthält, oder
wobei der Stabilisator Albumin oder Gelatine ist.

8. Diagnostische Zusammensetzung nach einem der Ansprüche 1 bis 7, ferner enthaltend einen oder mehrere Phospholipide, ausgewählt aus Phosphatidylserin, Phosphatidylethanolamin, Phosphatidylethanolcholin und Mischungen von Phospholipiden, die aus Kaninchenhirn extrahiert wurden.

9. Behälter, enthaltend die diagnostische Zusammensetzung nach einem der Ansprüche 1 bis 8.

10. Behälter nach Anspruch 9, wobei der Behälter (1) die Form eines Fläschchens oder einer Küvette aufweist und vorzugsweise so ausgebildet ist, dass sich das innere seitliche Profil von der Öffnung zum Boden verringert.

11. Behälter nach Anspruch 9 oder 10, dessen innerer Abschnitt so geformt ist, dass er an einer Vorrichtung zur Durchführung viskoelastischer Messungen angebracht werden kann, und vorzugsweise aus mindestens zwei verschiedenen Materialien besteht, wobei ein Material, das im Wesentlichen oder zumindest teilweise die äußere Form des Behälters bildet, eine hermetische Abdichtung des Behälters durch einen geeigneten Deckel ermöglicht, während das andere Material, das im Wesentlichen den Teil bedeckt, der die Testflüssigkeit enthält, eine ordnungsgemäße Haftung von Blut oder Blutbestandteilen ermöglicht, ohne die Gerinnungskaskade zu aktivieren.

12. Verfahren zur Durchführung einer viskoelastischen Analyse an einer Testflüssigkeit, ausgewählt aus Vollblut oder Blutplasma, umfassend die Schritte:
a) Bereitstellen einer Testflüssigkeit, ausgewählt aus Vollblut oder Blutplasma;
b) Bereitstellen eines Behälters (1) nach einem der Ansprüche 9 bis 11;
c) Hinzufügen der Testflüssigkeit in den Behälter (1), wodurch die darin enthaltene diagnostische Zusammensetzung aufgelöst wird;
d) gegebenenfalls Überführen der Mischung aus der Testflüssigkeit und der diagnostischen Zusammensetzung in eine Vorrichtung (4), die zur Durchführung einer viskolelastischen Analyse geeignet ist;
oder
Einbringen des Behälters 1 in eine Vorrichtung 4, die zur Durchführung einer viskolelastischen Analyse geeignet ist; und
e) Durchführen der viskoelastischen Analyse der Mischung.

13. Verfahren nach Anspruch 12, wobei die Testflüssigkeit vorzugsweise eine Blutprobe eines Säugetiers, besonders bevorzugt eines Menschen ist, und/oder
wobei Schritt c) etwa 1 bis 60 Sekunden, vorzugsweise etwa 2 bis 10 Sekunden, besonders bevorzugt etwa 5 Sekunden dauert, und/oder
wobei die Mischung in Schritt d) durch manuelles oder automatisches Pipettieren der Mischung aus dem Behälter (1) und durch Übertragen dadurch in die Vorrichtung (4) übertragen wird und/oder
wobei die Mischung in den Messbecher (2) der Vorrichtung (4) übertragen wird und/oder wobei die Vorrichtung (4) ein Thromboelastometer oder ein Thrombelastograph ist, und/oder
wobei die Analyse die Bestimmung der Gerinnungszeit, der Gerinnselbildungszeit, der Festigkeit des Gerinnsels über die Zeit und/oder Fibrinolyse umfasst.

14. Verwendung einer diagnostischen Zusammensetzung nach einem oder mehreren der Ansprüche 1 bis 8 oder eines Behälters (1) nach einem oder mehreren mehr der Ansprüche 9 bis 11 in einem Verfahren zur Analyse des viskoelastischen Verhaltens eines Testflüssigkeit ausgewählt aus Vollblut oder Blutplasma, wobei
vorzugsweise mindestens zwei diagnostische Zusammensetzungen von einem oder mehreren der Ansprüche 1 bis 8 oder mindestens zwei Behälter (1) von einem oder mehreren der Ansprüche 9 bis 11 in einem kombinierten Verfahren zur Analyse des viskoelastischen Verhaltens derselben Testflüssigkeit, ausgewählt aus Vollblut oder Blutplasma, verwendet werden.

## Revendications

1. Composition de diagnostic destinée à une utilisation dans l'analyse viscoélastique d'un liquide d'essai choisi parmi du sang complet ou du plasma sanguin, comprenant :
a) au moins un activateur de coagulation choisi à partir d'un facteur tissulaire (TF) ;
b) un sel de calcium, de préférence du CaCl₂, dans une quantité suffisante pour assurer une recalcification du liquide d'essai ; et
c) un ou plusieurs inhibiteurs d'héparine, de plaquettes ou de fibrinolyse ; **caractérisée en ce que**
les constituants sont présents dans une forme essentiellement sèche et dans une quantité suffisante pour effectuer une seule analyse viscoélastique d'un liquide d'essai spécifié,
et dans laquelle les constituants ne sont pas présents dans un mélange de substances, mais dans une forme séparée dans l'espace, dans laquelle les constituants sont chacun incorporés en pastilles séparées dans l'espace.

2. Composition selon la revendication 1, dans laquelle la séparation dans l'espace des constituants est réalisée en incorporant chaque constituant dans une matière de support séparée, dans laquelle la matière de support comprend de préférence au moins un hydrate de carbone, par exemple, du saccharose, du lactose ou de la cellulose.

3. Composition selon la revendication 2, dans laquelle la matière de support ne présente pas d'influence significative sur le comportement de coagulation, le comportement de formation d'un caillot, ou le comportement de lyse d'un caillot.

4. Composition de diagnostic selon la revendication 1, dans laquelle le facteur tissulaire est choisi parmi un TF lipidé ou un TF recombinant.

5. Composition de diagnostic selon la revendication 1, dans laquelle l'inhibiteur de plaquettes est un inhibiteur cyto-squelettique ou un antagoniste GPIIb/llla, par exemple, de la cytochalasine D, ou dans laquelle l'inhibiteur de fibrinolyse est choisi parmi l'aprotinine, l'acide tranéxamique ou l'acide epsilon amino caproïque eaca, ou dans laquelle l'inhibiteur d'héparine est choisi parmi l'héparinase, la protamine ou des peptides apparentés à la protamine.

6. Composition de diagnostic selon une ou plusieurs des revendications précédentes, dans laquelle le nouveau facteur de coagulation est choisi parmi un ou plusieurs facteurs de coagulation ou facteurs de coagulation activés, de préférence FXa ou FVa ou une protéine C ou FVIIa, ou
dans laquelle le CaCl₂ est présent dans une quantité d'environ 1 à 100 µmol/ml du liquide d'essai, de préférence, dans l'échantillon de sang, ou
dans laquelle la forme sèche est une forme lyophilisée.

7. Composition de diagnostic selon une ou plusieurs des revendications précédentes, qui comprend en outre un stabilisant, ou
dans laquelle le stabilisant est de l'albumine ou de la gélatine.

8. Composition de diagnostic selon l'une quelconque des revendications 1 à 7, comprenant en outre un ou plusieurs phospholipides choisis parmi la phosphatidylsérine, la phosphatidyléthanolamine, la phosphatidyléthanolcholine et des mélanges de phospholipides extraits du cerveau de lapin.

9. Récipient comprenant la composition de diagnostic selon une ou plusieurs des revendications 1 à 8.

10. Récipient selon la revendication 9, ledit récipient (1) prenant la forme d'une fiole ou d'une cuvette, et étant formé de préférence de manière à ce que le profil latéral intérieur se réduit à partir de l'orifice jusqu'au fond.

11. Récipient selon la revendication 9 ou la revendication 10, avec sa partie intérieure formée de manière à ce qu'il puisse être fixé à un dispositif pour effectuer des mesures viscoélastiques, et étant de préférence constitué d'au moins deux matières différentes, dans lequel une matière formant de base ou au moins partiellement la forme extérieure du récipient permet une fermeture hermétique du récipient par un couvercle approprié, tandis que l'autre matière recouvrant de base la partie constituant le liquide d'essai permet une adhésion propre du sang ou des composants sanguins sans activer la coagulation en cascade.

12. Procédé de réalisation d'une analyse viscoélastique sur un liquide d'essai choisi parmi du sang complet ou du plasma sanguin, comprenant les étapes :
a) de fourniture d'un liquide d'essai choisi parmi du sang complet ou du plasma sanguin ;
b) de fourniture d'un récipient (1) selon l'une des revendications 9 à 11 ;
c) d'ajout du liquide d'essai dans ledit récipient (1), en dissolvant ainsi la composition de diagnostic y étant contenue ;
d) de transfert éventuel du mélange dudit liquide d'essai et de ladite composition de diagnostic dans un appareil (4) approprié pour effectuer une analyse viscoélastique ;
ou
de disposition du récipient 1 dans un appareil 4 approprié pour effectuer une analyse viscoélastique ; et
e) d'exécution de l'analyse viscoélastique dudit mélange.

13. Procédé selon la revendication 12, dans lequel le liquide d'essai est de préférence un échantillon sanguin de mammifère, plus préférentiellement d'un humain, et/ou
dans lequel l'étape c) dure environ de 1 à 60 secondes, de préférence environ de 2 à 10 secondes, plus préférentiellement environ 5 secondes, et/ou
dans lequel le mélange est transféré dans l'étape d) en pipetant manuellement ou automatiquement le mélange à partir du récipient (1) et en le transférant ainsi vers l'appareil (4), et/ou dans lequel le mélange est transféré de la coupe de mesure (2) de l'appareil (4), et/ou dans lequel l'appareil (4) est un thromboélastomètre ou un thromboélastographe, et/ou dans lequel l'analyse comprend la détermination du temps de coagulation, du temps de formation du caillot, de la solidité du caillot dans le temps et/ou de la fibrinolyse.

14. Utilisation d'une composition de diagnostic selon une ou plusieurs des revendications 1 à 8 ou d'un récipient (1) selon une ou plusieurs des revendications 9 à 11 dans un procédé d'analyse du comportement viscoélastique d'un liquide d'essai choisi parmi du sang complet ou du plasma sanguin, dans laquelle,
de préférence, au moins deux compositions de diagnostic selon une ou plusieurs des revendications 1 à 8, ou au moins deux récipients (1) selon une ou plusieurs des revendications 9 à 11, sont utilisés dans un procédé combiné pour analyser le comportement viscoélastique du même liquide d'essai choisi parmi le sang complet ou le plasma sanguin.
